# EUROPÄISCHE PATENTANMELDUNG

(11) **EP 3 671 211 A1**
(43) Veröffentlichungstag der Anmeldung: **24.06.2020**
(21) Anmeldenummer: 18215582.0
(22) Anmeldetag: 21.12.2018
(51) Int. Cl.: G01N 33/558

(54) **VORRICHTUNG UND VERFAHREN ZUM VISUELLEN NACHWEIS VON ANALYTEN IN EINER SPEICHELPROBE**

(71) Anmelder: Protzek Gesellschaft für Biomedizinische Technik GmbH, 4133 Prattein (CH)
(72) Erfinder: PROTZEK, Christoph, 79539 Lörrach (DE)
(74) Vertreter: Patentanwälte Dörner & Kötter PartG mbB

(57) **Zusammenfassung**

Die Erfindung betrifft eine Vorrichtung zum visuellen Nachweis von Analyten in einer vom menschlichen oder tierischen Organismus stammenden Speichelprobe, umfassend eine Trägerplatte (1) mit wenigstens einer Teststreifenaufnahme (12), in der ein mit einem Probeaufnahmeabschnitt (32) und einem Testabschnitt (34) versehener Teststreifen (3) angeordnet ist, welche wenigstens eine Teststreifenaufnahme (12) einen Probenaufgabeabschnitt (13) aufweist und zumindest bereichsweise über eine Deckschicht verschlossen ist, die im Bereich des Testabschnitts (34) des Teststreifens (3) der wenigstens einen Teststreifenaufnahme (12) ein Kontrollfenster sowie im Bereich des Probenaufgabeabschnitts ein Probeaufgabefenster (14) aufweist, dadurch gekennzeichnet, dass das Probeaufgabefenster (14) zur Aufnahme eine Speichelprobenschwamms ausgebildet ist, wobei Mittel zum Auspressen eines auf dem Probeaufgabefenster (14) positionierten Speichelprobenschwamms angeordnet sind. Die Erfindung betrifft weiterhin ein Verfahren zur Analyse einer Speichelprobe mit einer solchen Vorrichtung.

## Beschreibung

Die Erfindung betrifft eine Vorrichtung zum visuellen Nachweis von Analyten in einer vom menschlichen oder tierischen Organismus stammenden Speichelprobe nach dem Oberbegriff des Patentanspruchs 1. Die Erfindung betrifft weiterhin ein Verfahren zum visuellen Nachweis von Analyten in einer Speichelprobe nach dem Patentanspruch 14.

Vorrichtungen zum Nachweis von Analyten in einer flüssigen Probe, zum Beispiel von Drogen, Medikamenten oder Antikörpern als Zeichen für bestimmte Erkrankungen in einer Körperflüssigkeit oder Feststellungen im Sinne von Konzentrationsangaben sind in unterschiedlichen Ausführungen bekannt. Besonderes Interesse besteht an solchen Geräten, die auch außerhalb eines Labors eingesetzt werden können. Diese müssen tragbar und leicht zu bedienen sein sowie in kurzer Zeit ein zuverlässiges Ergebnis liefern. Insbesondere zur Durchführung von Tests auf Drogenkonsum oder Medikamentenmissbrauch oder dergleichen durch die Polizei ist eine Vorrichtung erforderlich, die auch im Freien bei unterschiedlichen wechselnden Lichtverhältnissen, Wetterverhältnissen und Temperaturen einen derartigen Test ermöglicht.

In der DE 20 2014 002 369 U1 wird hierzu ein Gerät vorgeschlagen, in das eine Testkassette einführbar ist und dass mit einer Lichtquelle versehen ist, mittels derer die untere Wand der aus einem lichtdurchlässigem Kunststoff bestehenden Testkassette sowie den Testbereich des in der Testkassette angeordneten Teststreifens durchstrahlt und das Testergebnis ausleuchtet. Die Testkassette ist zur Durchführung eines lateral flow immuno-assays, Aptamer basierter Assay oder Encym immuno assay (EIA) ausgelegt und besteht aus einem unteren Deckel und einem oberen Deckel. Eingeschlossen von diesen beiden Deckeln enthält die Testkassette einen Teststreifen, der innerhalb einer Aufnahme in Position gehalten wird. In dem oberen Deckel befindet sich über dem Probenaufnehmer des Teststreifens eine Einfüllöffnung, durch die eine zu untersuchende flüssige Probe eingegeben werden kann. Über den mittleren Testbereich des Teststreifens befindet sich ein Kontrollfenster, durch welches das Testergebnis abgelesen werden kann. Derartige Kassetten haben sich im mobilen Einsatz bewährt. Nachteilig an den vorbekannten Kassetten ist jedoch, dass dessen Herstellung aufwendig ist und diese relativ viel Platz benötigen.

Zur Lösung dieser Problematik wird in der DE 20 2016 104 645 U1 hierzu eine Vorrichtung vorgeschlagen, die eine im Wesentlichen aus Pappe hergestellte Trägerschicht umfasst, die mit einer Deckfolie versehen ist und bei der Teststreifenaufnahmen und Reservoire durch Einprägungen oder durch Ausnehmungen einer zwischen Deckfolie und Trägerplatte angeordneten Zwischenschicht ausgebildet sind. Diese Vorrichtung zeichnet sich besonders dadurch aus, dass sie sehr leicht und platzsparend, günstig herstellbar und darüber hinaus umweltfreundlich zu entsorgen ist.

Voraussetzung für die Funktion der bei den vorgenannten Vorrichtungen zum Einsatz kommenden Teststreifen ist die Aufgabe einer fließfähigen Probenflüssigkeit in ausreichender Menge.

Zum qualitativen Nachweis von Drogen im menschlichen Speichel sind Speicheltests in unterschiedlichen Ausführungen bekannt. Dabei wird der Speichel zunächst dem Probanden mittels eines Schwamms aus der Mundhöhle entnommen und nachfolgend einem immunchromatographischen Teststreifen zugeführt ("Lateral Flow" - Technologie). Da Drogenkonsumenten aufgrund der physiologischen Wirkung der Drogen häufig einen sehr trockenen Mund haben, steht nur wenig Speichel für die Probennahme zur Verfügung. Zur Durchführung des Tests wird dieser Speichel einer Pufferflüssigkeit zugegeben, welche sodann auf den Teststreifen aufgegeben wird. Nachteilig an diesem Verfahren ist, dass durch die Verdünnung der Speichelprobe eine Senkung der Konzentration des Analyten in der Probe erfolgt, wodurch der eigentliche Test beeinträchtigt ist. Andere Testverfahren erweisen sich als komplex und aufwändig bedienbar, wodurch sie für den mobilen Einsatz beispielsweise bei der Verkehrspolizei ungeeignet erscheinen.

Hier will die Erfindung Abhilfe schaffen. Der Erfindung liegt die Aufgabe zugrunde, eine Vorrichtung zum visuellen und messtechnischen Nachweis von Analyten in einer Speichelprobe bereitzustellen, die einfach aufgebaut ist, für den mobilen Einsatz geeignet ist und die auch bei Vorliegen einer geringen Probenmenge ein unverfälschtes Testergebnis ermöglicht. Gemäß der Erfindung wird diese Aufgabe durch eine Vorrichtung mit den Merkmalen des Patentanspruchs 1 gelöst.

Mit der Erfindung ist eine Vorrichtung zum visuellen und messtechnischen Nachweis von Analyten in einer Speichelprobe bereitgestellt, die einfach aufgebaut ist, für den mobilen Einsatz geeignet ist und die auch bei Vorliegen einer geringen Probenmenge ein unverfälschtes Testergebnis ermöglicht. Durch das Vorsehen von Mitteln zum Auspressen eines auf dem Probeaufgabefenster positionierten Speichelprobenschwamms ist die Ausbringung einer maximalen Speichelprobenmenge auf den Teststreifen ermöglicht.

In Weiterbildung der Erfindung umfassen die Mittel zum Auspressen eines auf dem Probeaufgabefenster positionierten Speichelprobenschwamms eine schwenkbar mit der Trägerplatte verbundene Druckplatte. Hierdurch ist eine integrierte und zugleich platzsparende und hygienische Auspressvorrichtung erzielt.

In Ausgestaltung der Erfindung weist die Druckplatte im Kontaktbereich mit dem Probeaufgabefenster eine Auswölbung aus. Hierdurch ist der Auspressdruck auf den Speichelprobenschwamm maximiert.

In weiterer Ausgestaltung der Erfindung ist die Teststreifenaufnahme im Bereich des Probeaufgabefensters mulden- oder taschenförmig ausgebildet. Hierdurch ist eine maximale Ausbringung der Speichelprobe aus einem eingelegten Speichelprobenschwamm unterstützt.

In weiterer Ausgestaltung der Erfindung sind wenigstens zwei Teststreifenaufnahmen angeordnet, die in einem gemeinsamen Probeaufgabenabschnitt münden. Hierdurch ist eine gleichzeitige Probenbeaufschlagung von zwei Teststreifen ermöglicht.

In Weiterbildung der Erfindung ist dem Probeaufgabefenster vorgelagert ein Trägerflüssigkeitsaufgabefenster angeordnet. Hierdurch ist die Aufgabe einer Trägerflüssigkeit hinter der Speichelprobe ermöglicht. Durch die Kapillarwirkung des Teststreifens wird die Trägerflüssigkeit hinter der Speichelprobe durch den Teststreifen bewegt und "schiebt" somit die (geringe) Speichelprobe durch den Teststreifen, ohne diese mit der Trägerflüssigkeit zu vermischen. Auf diese Weise ist auch mit einer mengenmäßig sehr geringen Speichelprobe, die im Stand der Technik die Zugabe einer Flüssigkeit bedarf, um den Test "zum Laufen" zu bringen, ein zuverlässiger Test ohne eine die Analytkonzentration beeinträchtigenden Vermischung mit einer Flüssigkeit ermöglicht.

In Ausgestaltung der Erfindung ist das Trägerflüssigkeitsaufgabefenster über eine zumindest bereichsweise abziehbare Folie verschlossen. Hierdurch ist einer Kontamination des Teststreifens entgegengewirkt. Bevorzugt ist auch das Probeaufgabefenster über eine zumindest bereichsweise abziehbare Folie verschlossen.

In weiterer Ausgestaltung der Erfindung weist die Trägerplatte im Bereich des Trägerflüssigkeitsaufgabefensters ein Trägerflüssigkeitsreservoir auf. Hierdurch ist eine gleichmäßige Zuführung von Trägerflüssigkeit hinter der Speichelprobe zu deren "Transport" durch den Teststreifen ermöglicht. Dabei ist bevorzugt zwischen Deckfolie und Teststreifen ein Trägerflüssigkeitsaufnahmevlies angeordnet.

In weiterer Ausgestaltung der Erfindung ist das Probeaufgabefenster über eine zumindest bereichsweise abziehbare Folie verschlossen. Hierdurch ist einer Kontamination der Teststreifen vor deren Benutzung verhindert.

In Weiterbildung der Erfindung ist die Trägerplatte aus Pappe hergestellt, wobei vorhandene Teststreifenaufnahmen und/oder Trägerflüssigkeitsreservoire durch Einprägungen ausgebildet sind. Hierdurch ist eine umweltfreundliche und zugleich platzsparende Gestaltung der Vorrichtung erzielt, die zudem kostengünstig herstellbar ist.

In Ausgestaltung der Erfindung ist die wenigstens eine Teststreifenaufnahme zumindest bereichsweise über eine nicht lösbar mit der Trägerplatte verbundene, insbesondere mit dieser verklebten Deckfolie verschlossen. Hierdurch ist ein Schutz der aufgenommenen Teststreifen gegenüber äußeren Einflüssen erzielt.

In weiterer Ausgestaltung der Erfindung ist die Druckplatte aus Pape hergestellt und über eine Knickfalz mit der Trägerplatte verbunden. Hierdurch ist eine einteilige, umweltfreundliche und zugleich kostengünstig herstellbare Vorrichtung erzielt. Dabei ist die Knickfalz bevorzugt derart ausgebildet, dass ein Verschwenken der Druckplatte um etwa 180 Grad ermöglicht ist. Hierdurch kann die Druckplatte an die Oberseite (Auspressposition) sowie an die Unterseite der Trägerplatte (Ausleseposition) angelegt werden, wodurch eine Verwendung von Optischen Auslesegeräten ermöglicht ist.

Vorzugsweise ist die Auswölbung durch eine Einprägung gebildet. Hierdurch ist eine kostengünstige Herstellung erzielt.

In einer weiteren Ausgestaltung der Erfindung ist ein mit einem Speichelprobeschwamm versehenes Probestäbchen angeordnet, das aus Pappe gebildet ist. Hierdurch ist ein umweltgerecht entsorgbares, platzsparendes Speichelprobenanalyseset gebildet.

Bevorzugt ist das Probestäbchen über eine Reißlinie, insbesondere eine Perforationslinie mit der Trägerplatte oder der Druckplatte verbunden. Hierdurch ist eine kostengünstige, einteilige Herstellung des Speichelprobenanalysesets ermöglicht, wobei das Probestäbchen zugleich verliersicher mit der Vorrichtung verbunden ist. Unter einer Reißlinie ist vorliegend eine linienförmige Sollbruchstelle zu verstehen, die eine definierte Abtrennung des Probestäbchens von der Trägerplatte bzw. der Druckplatte ohne das Erfordernis eines Trennwerkzeugs ermöglicht.

Der vorliegenden Erfindung liegt die weiterhin die Aufgabe zugrunde, ein Verfahren zum visuellen und messtechnischen Nachweis von Analyten in einer Speichelprobe bereitzustellen, das den mobilen Einsatz geeignet ist und das auch bei Vorliegen einer geringen Probenmenge ein unverfälschtes Testergebnis ermöglicht. Gemäß der Erfindung wird diese Aufgabe durch ein Verfahren mit den Merkmalen des Patentanspruchs 14 gelöst.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:
- Figur 1: die schematische Darstellung einer Vorrichtung zum visuellen Nachweis von Analyten in einer flüssigen Probe;
- Figur 2: die schematische Darstellung eines Teststreifens der Vorrichtung aus Figur 1 und
- Figur 3: die schematische Darstellung einer Vorrichtung zum visuellen Nachweis von Analyten in einer flüssigen Probe in einer weiteren Ausführungsform.

Die als Ausführungsbeispiel gewählte Vorrichtung zum visuellen Nachweis von Analyten in einer Speichelprobe besteht im Wesentlichen aus einer Trägerplatte 1, die entlang ihrer Längsseite schwenkbar mit einer Deckplatte 2 verbunden ist. Trägerplatte 1 und Deckplatte 2 sind im Ausführungsbeispiel aus Pappe hergestellt und aus einem einzigen mittig mit einer Knickfalz 11 versehenen Kartonzuschnitt gebildet. Auf der Trägerplatte 11 sind zwei Teststreifenaufnahmen 12 eingebracht, die jeweils einen Teststreifen 3 aufnehmen und die mit einer transparenten, flüssigkeitsdichten Deckfolie versehen sind, über welche die Teststreifen 3 in den Teststreifenaufnahmen 12 fixiert sind. Die Teststreifenaufnahmen 12 sind im Ausführungsbeispiel in die Trägerplatte 1 eingeprägt.

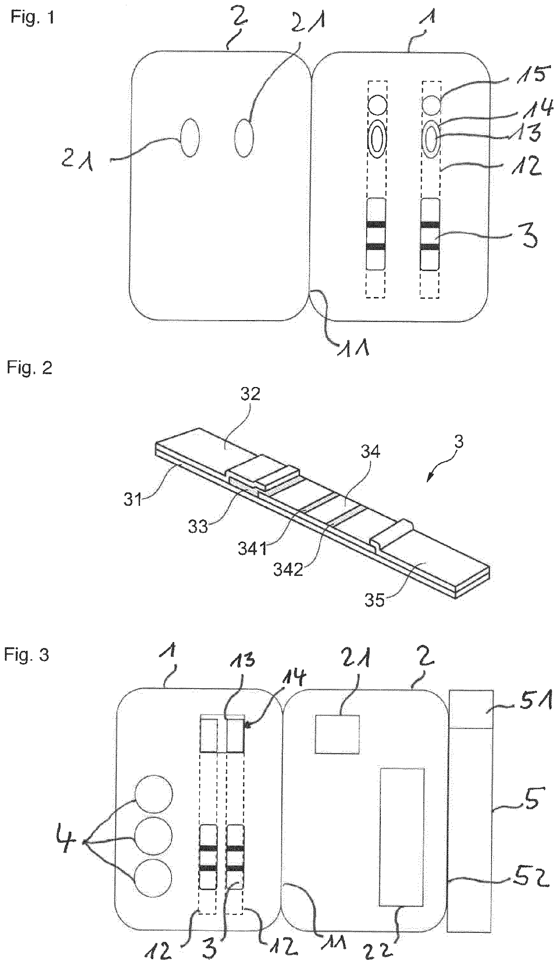

Der Teststreifen 3 ist in Figur 2 im Stufenschnitt dargestellt. Der Teststreifen 3 weist eine Trägerfolie 31 auf, auf der an einem Ende ein Probeaufnahmeabschnitt 32 angeordnet ist. Unmittelbar benachbart und teilweise unter dem Probeaufnahmeabschnitt 32 befindet sich auf der Trägerfolie 31 ein Antikörperbelagabschnitt 33, der Antikörper enthält, die mit Goldpartikeln konjugiert sind und die zu einem festzustellenden Analyten bzw. zu einem Antigen dieses Analyten passen und in eine immunologische Reaktion treten können. An den Antikörperbelagabschnitt 33 schließt sich ein Testabschnitt 34 an, der in Form einer Membran ausgebildet ist, auf den sich beabstandet zueinander eine Testlinie 341 und eine Kontrolllinie 342 befinden, die durch in einem Reagenz für den Nachweis eines bestimmten Analyten enthaltenen Antigen ausgebildet sind und vor Aufgabe einer Probe unsichtbar sind.

In Höhe des Probenaufnahmeabschnitts 32 eines jeden Teststreifens 3 weist die Teststreifenaufnahme 12 einen muldenförmig ausgebildeten Probenaufgabeabschnitt 13 auf. In diesem Bereich ist in der Deckfolie ein Probeaufgabefenster 14 angeordnet, das durch eine in die Deckfolie eingebrachte Ausnehmung gebildet ist und das mit einer - nicht dargestellten - abziehbaren Abdeckfolie verschlossen ist. Das Probeaufgabefenster 14 dient gemeinsam mit dem muldenförmigen Probenaufgabeabschnitt 13 der Teststreifenaufnahme 12 der Aufnahme eines Speichelprobenschwamms.

In Fließrichtung des Testreifens 3 gesehen vor dem Probeaufgabefenster 14 ist ebenfalls im Bereich des Probenaufnahmeabschnitts 32 eines jeden Teststreifens 3 ein Trägerflüssigkeitsaufgabefenster 15 angeordnet. Auch das Trägerflüssigkeitsaufgabefenster 15 ist im Ausführungsbeispiel durch eine in die Deckfolie eingebrachte Ausnehmung gebildet und über eine - nicht dargestellte - abziehbare Abdeckfolie verschlossen.

Den Probeaufgabefenstern 14 gegenüberliegend sind in der Deckplatte 2 zwei Auswölbungen 21 angeordnet, die im zusammengeklappten Zustand der Vorrichtung in die hinter den Probeaufgabefenstern 14 angeordneten muldenförmigen Probeaufgabeabschnitte13 der Teststreifenaufnahmen 12 eingreifen. Die Auswölbungen 21 dienen dem Auspressen von in einem eingelegten Speichelprobenschwamm befindlichen Speichel, nachdem dieser in das zugeordnete Probeaufgabefenster 14 eingelegt worden ist.

Bei der Durchführung eines lateral flow assays zeigt die Testlinie 341 das Ergebnis des Tests an; die Kontrolllinie 342 gibt an, ob eine ausreichende Probemenge aufgegeben worden ist, also ob der Test valide ist und seine Durchführung sachgemäß erfolgte. An seinem dem Probenaufnahmeabschnitt 32 gegenüberliegenden Ende ist an den Teststreifen 3 ein Absorberabschnitt 35 angeordnet.

Vor der Durchführung eines Tests sind keine Linien 341, 342 ersichtlich. Bei der Durchführung eines Tests wird ein mit einer Speichelprobe versehener Speichelprobenschwamm in ein Probeaufgabefenster 14 eingelegt und durch Zuklappen der Deckplatte 2 über die Auswölbung 21 ausgepresst. Hierdurch gelang die Speichelprobe auf den Probenaufnahmeabschnitt 32 eines Teststreifens 3. Durch Kapillarwirkung fließt die Probenflüssigkeit in Richtung Testabschnitt 34. Sie kommt dabei zunächst mit dem Antikörperbelagabschnitt 33 in Kontakt, in dem sich mit Goldpartikeln konjugierte Antikörper zum Antigen eines festzustellenden Analyten in standardisierter (dosierter) Menge befinden. Die Antikörper weisen eine für verschiedene Analyten (Drogen) unterschiedliche Bindungsaktivität auf. Die mit Goldpartikeln konjugierten Antikörper werden durch die Probenflüssigkeit mitgenommen, welche den Testabschnitt 34 durchfließt. Enthält die Probenflüssigkeit nicht den festzustellenden Analyten, so werden beim Durchfließen des Testsabschnitts 34 die dort im Bereich der Testlinie 341 befindlichen Antigene des Analyten durch die von der Probenflüssigkeit mitgenommenen Antikörper gebunden und es bildet sich eine sichtbare Testlinie 341 heraus, die durch die mit den Antikörpern konjugierten Goldpartikel eine rote oder braune Farbe erhält. Enthält die Probenflüssigkeit den festzustellenden Analyten, so werden dessen Antigene bereits durch die Antikörper im Bereich des Antikörperbelagabschnitts 33 des Teststreifens 3 gebunden und es kann im Testabschnitt 34 nicht mehr zu einer Anlagerung kommen, weshalb sich keine sichtbare Testlinie 341 herausbildet. Die Kontrolllinie 342 zeigt an, ob der Test valide ist. In Figur 2 ist die Testlinie 341 farbig herausgebildet, weshalb der Test in Bezug des entsprechenden Analyten negativ ist.

Läuft die Speichelprobe mangels Menge nicht ausreichend in Richtung Testabschnitt 34, so wird in das Trägerflüssigkeitsaufgabefenster 15 auf den darunter befindlichen Probenaufnahmeabschnitt 32 des Teststreifens 3 eine geringe Menge einer Trägerflüssigkeit, beispielsweise ein Tropfen destilliertes Wasser, aufgegeben. Diese Trägerflüssigkeit fließt durch Kapillarwirkung in Richtung Testabschnitt 34 und schiebt dabei die Speichelprobe vor sich her, ohne sich jedoch mit dieser zu vermischen.

Die erfindungsgemäße Vorrichtung zeichnet sich dadurch aus, dass sie bei einer kompakten Ausbildung unter Einsatz geringer Probemengen, insbesondere Speichelprobemengen die Durchführung eines validen Tests ermöglicht. Durch die erfindungsgemäße Zugabe einer Trägerflüssigkeit, die hinter die Speichelprobe in das Kapillarsystem des Teststreifens 3 gegeben wird, ist ein Transport einer geringen Probemenge durch den Teststreifen 3 ermöglicht, ohne das eine Vermischung der Trägerflüssigkeit mit der Probe erfolgt.

Durch die Herstellung der Vorrichtung im Wesentlichen aus Kartonwerkstoffen ist zudem eine umweltgerechte Entsorgung dieser zur einmaligen Verwendung bestimmten Vorrichtung ermöglicht.

In Figur 3 ist eine weitere Ausgestaltung der erfindungsgemäßen Vorrichtung zum visuellen Nachweis von Analyten in einer Speichelprobe gezeigt. Hier sind auf der Trägerplatte 1 parallel zueinander zwei Teststreifenaufnahmen 12 angeordnet, die in einem gemeinsamen Probeaufgabeabschnitt 13 münden und die jeweils einen Teststreifen 3 aufnehmen und die wiederum über eine gemeinsame Deckfolie verschlossen sind. In die Deckfolie ist über dem gemeinsamen Probeaufgabeabschnitt ein Probeaufgabefenster 14 eingebracht, das über eine - nicht dargestellt - Abziehfolie verschlossen ist. Die Teststreifenaufnahmen 12 und der Probeaufnahmeabschnitt 13 sind wiederum in die aus Pappe hergestellte Trägerplatte 1 eingeprägt. Neben den Teststreifenaufnahmen sind auf der Trägerplatte 1 beabstandet zueinander drei Dried Blod Spots 4 angeordnet, die über die Deckfolie auf der Trägerplatte 1 fixiert sind, wobei die Deckfolie im Bereich eines jeden Dried Blod Spot 4 jeweils ein Fenster aufweist, das über eine - nicht dargestellte - abziehbare Abdeckfolie verschlossen ist.

Dem gemeinsamen Probeaufgabefenster 14 gegenüberliegend ist in der Deckplatte 2 eine Auswölbung 21 angeordnet, die im zusammengeklappten Zustand der Vorrichtung in den hinter dem Probeaufgabefenster 14 angeordneten muldenförmigen gemeinsamen Probeaufgabeabschnitte13 der Teststreifenaufnahmen 12 eingreift. Die Auswölbung 21 dient wiederum dem Auspressen von in einem eingelegten Speichelprobenschwamm befindlichen Speichel, nachdem dieser in das zugeordnete Probeaufgabefenster 14 eingelegt worden ist.

Beabstandet zu der Auswölbung 21 ist in die Deckplatte 2 ein DS-Fenster 22 eingebracht, das im zusammengeklappten Zustand, in dem die Deckplatte 2 auf der Trägerplatte 1 aufliegt, die drei Dried Blod Spots 4 einrahmt. Seitlich ist an die Deckplatte 2 ein Probenstäbchen 5 angeordnet, das in Form eines Pappstreifens ausgebildet ist, das mit einem Speichelschwamm 51 versehen ist. Das Probestäbchen ist über eine Perforationslinie 52 mit der Deckplatte 2 verbunden.

In dieser Ausführungsform sind Trägerplatte 1, Deckplatte 2 und Probenstäbchen 5 aus einem gemeinsamen Pappzuschnitt gebildet, wobei Trägerplatte 1 und Deckplatte 2 über eine Knickfalz 11 sowie Deckplatte 2 und Probenstäbchen 5 über eine Reißlinie, vorliegend eine Perforationslinie 52 verbunden sind. Die Teststreifenaufnahmen 12 und die Auswölbung 21 sind durch Einprägungen in dem Pappzuschnitt gebildet. Nach Aufbringung der Teststreifen 3 sowie der Dried Blod Spots 4 sind diese durch eine den gesamten Pappzuschnitt überdeckende, als Klebefolie ausgebildete Deckfolie fixiert. Hierdurch ist eine sehr kostengünstige Herstellung ermöglicht.

## Patentansprüche

1. Vorrichtung zum visuellen Nachweis von Analyten in einer vom menschlichen oder tierischen Organismus stammenden Speichelprobe, umfassend eine Trägerplatte (1) mit wenigstens einer Teststreifenaufnahme (12), in der ein mit einem Probeaufnahmeabschnitt (32) und einem Testabschnitt (34) versehener Teststreifen (3) angeordnet ist, welche wenigstens eine Teststreifenaufnahme (12) einen Probenaufgabeabschnitt (13) aufweist und zumindest bereichsweise über eine Deckschicht verschlossen ist, die im Bereich des Testabschnitts (34) des Teststreifens (3) der wenigstens einen Teststreifenaufnahme (12) ein Kontrollfenster sowie im Bereich des Probenaufgabeabschnitts ein Probeaufgabefenster (14) aufweist, **dadurch gekennzeichnet, dass** das Probeaufgabefenster (14) zur Aufnahme eine Speichelprobenschwamms ausgebildet ist, wobei Mittel zum Auspressen eines auf dem Probeaufgabefenster (14) positionierten Speichelprobenschwamms angeordnet sind.

2. Vorrichtung nach Anspruch 1, **dadurch gekennzeichnet, dass** die Mittel zum Auspressen eines auf dem Probeaufgabefenster (14) positionierten Speichelprobenschwamms eine schwenkbar mit der Trägerplatte (1) verbundene Druckplatte (2) umfassen, die im Kontaktbereich mit dem Probeaufgabefenster (14) eine Auswölbung (21) aufweist.

3. Vorrichtung nach einem der vorgenannten Ansprüche, **dadurch gekennzeichnet, dass** der Probenaufgabenabschnitt (13) der Teststreifenaufnahme (12) mulden- oder taschenförmig ausgebildet ist.

4. Vorrichtung nach einem der vorgenannten Ansprüche, **dadurch gekennzeichnet, dass** wenigstens zwei Teststreifenaufnahmen (12) angeordnet sind, die in einem gemeinsamen Probenaufgabenabschnitt (13) münden.

5. Vorrichtung nach einem der vorgenannten Ansprüche, **dadurch gekennzeichnet, dass** dem Probeaufgabefenster (14) vorgelagert ein Trägerflüssigkeitsaufgabefenster (15) angeordnet ist.

6. Vorrichtung nach Anspruch 5, **dadurch gekennzeichnet, dass** das Trägerflüssigkeitsaufgabefenster (15) über eine zumindest bereichsweise abziehbare Folie verschlossen ist.

7. Vorrichtung nach Anspruch 5 oder 6, **dadurch gekennzeichnet, dass** die Trägerplatte (1) im Bereich des Trägerflüssigkeitsaufgabefensters (15) ein Trägerflüssigkeitsreservoir aufweist.

8. Vorrichtung nach Anspruch 7, **dadurch gekennzeichnet, dass** zwischen Deckfolie und Teststreifen (3) ein Trägerflüssigkeitsaufnahmevlies angeordnet ist.

9. Vorrichtung nach einem der vorgenannten Ansprüche, **dadurch gekennzeichnet, dass** das Probeaufgabefenster (14) über eine zumindest bereichsweise abziehbare Folie verschlossen ist.

10. Vorrichtung nach einem der vorgenannten Ansprüche, **dadurch gekennzeichnet, dass** die Trägerplatte (1) aus Pappe hergestellt ist, wobei vorhandene Teststreifenaufnahmen (12) und/oder Trägerflüssigkeitsreservoire bevorzugt durch Einprägungen ausgebildet sind.

11. Vorrichtung nach Anspruch 10, **dadurch gekennzeichnet, dass** die wenigstens eine Teststreifenaufnahme (12) zumindest bereichsweise über eine nicht lösbar mit der Trägerplatte (1) verbundene, insbesondere mit dieser verklebte Deckfolie verschlossen ist.

12. Vorrichtung nach Anspruch 10 oder 11, **dadurch gekennzeichnet, dass** die Druckplatte (2) aus Pape hergestellt ist und über eine Knickfalz (11) mit der Trägerplatte (1) verbunden ist, wobei die Auswölbung (21) vorzugsweise durch eine Einprägung gebildet ist.

13. Vorrichtung nach einem der Ansprüche 10 bis 12, **dadurch gekennzeichnet, dass** ein mit einem Speichelprobeschwamm (51) versehenes Probestäbchen (5) angeordnet ist, das aus Pappe gebildet ist und das bevorzugt über eine Reißlinie, insbesondere eine Perforationslinie (51) mit der Trägerplatte (1) oder der Druckplatte (2) verbunden ist.

14. Verfahren zur Analyse einer Speichelprobe, insbesondere mit eine Vorrichtung nach einem der vorgenannten Ansprüche, wobei zunächst ein mit der Speichelprobe versehener Speichelprobenschwamm an einer Stelle über dem Probenaufnahmeabschnitt (32) eines Teststreifens (3) ausgepresst wird und nachfolgend in Fließrichtung gesehen vor der Speichelprobeaufgabestelle eine Menge an Trägerflüssigkeit auf den Probenaufnahmeabschnitt (32) des Teststreifens (3) gegeben wird.
